# EUROPEAN PATENT APPLICATION

(11) **EP 4 378 576 A1**
(43) Date of publication of application: **05.06.2024**
(21) Application number: 22210271.7
(22) Date of filing: 29.11.2022
(51) Int. Cl.: B01J 19/26, B01J 4/00, C07C 263/10, C07C 265/14

(54) **APPARATUS AND PROCESS FOR PREPARING ISOCYANATES**

(71) Applicant: BASF SE, 67056 Ludwigshafen am Rhein (DE)
(72) Inventor: MAIXNER, Stefan, 68723 Schwetzingen (DE); KUD, Alexander, 67705 Trippstadt (DE); LIPP, Stefan, 67056 Ludwigshafen am Rhein (DE); REINDEL, Klaus, 67056 Ludwigshafen am Rhein (DE); WON, Myung Un, 59616 Yeosu (KR); PARK, Man Gyun, 59610 Yeosu (KR); PARAKADAVIL, Shah, 67056 Ludwigshafen am Rhein (DE)
(74) Representative: BASF IP Association

(57) **Abstract**

The invention relates to an apparatus and a process for preparing isocyanates by reacting the corresponding amines with phosgene in the liquid phase, wherein either the amine as a first reactant and the phosgene as a second reactant or the phosgene as a first reactant and the amine as a second reactant are mixed in a mixing chamber (1) to form a reaction mixture and the reaction mixture is fed to a reactor, the first reactant being added through a coaxial inlet (3) penetrating a back plane (2) of the mixing chamber (1) and extending coaxially into the mixing chamber (1) with an orifice (4) opening out into the mixing chamber (1) at a distance L1 to the back plane (2), and the second reactant being added through radial orifices (5) opening out into the mixing chamber (1) in at least a first plane (6) arranged perpendicular to the axis (8) of the mixing chamber (1), wherein all radial orifices (5) are arranged upstream of the orifice (4) of the coaxial inlet (3) in the main flow direction of the reaction mixture.

## Description

The invention relates to an apparatus and a process for preparing isocyanates by reacting the corresponding amines with phosgene in the liquid phase, the apparatus comprising a mixing chamber for mixing the amine and the phosgene to form a reaction mixture, a coaxial inlet penetrating a back plane of the mixing chamber and extending coaxially into the mixing chamber with an orifice opening out into the mixing chamber at a distance to the back plane, and radial orifices opening out into the mixing chamber in at least a first plane arranged perpendicular to the axis of the mixing chamber.

Isocyanates can be prepared by phosgenating the corresponding amines, in principle by a liquid phase or gas phase phosgenation. Liquid phase phosgenation is characterised by the fact that the reaction can be carried out at lower temperatures than gas phase phosgenation and no evaporation of the reactants is required.

In liquid phase phosgenation, an amine-containing reactant stream is fed in the liquid phase and mixed with a phosgene-containing reactant stream. The phosgene may be dissolved in an inert solvent. The phosgene-containing reactant stream is sprayed into a mixing chamber in which it is mixed with the amine-containing reactant stream. The amine and the phosgene react with release of hydrogen chloride (HCl) to give the corresponding isocyanates.

Rapid mixing of the amine with the phosgene is necessary, since the isocyanate formed, in the case of too low a phosgene concentration, reacts with the excess amine to give urea or other troublesome, high-viscosity and solid by-products. For this reason, rapid mixing and a short residence time in the reaction chamber are required.

Several approaches aiming at reducing the residence time, in particular in the mixing zone, have been developed and are known from the prior art. In one approach, the reactants are injected perpendicular to each other in a mixing chamber. The documents DD 300 168 A7 and WO 2010/015667 A1 for example show an apparatus in which amine and phosgene are first mixed in a mixing chamber to give a reaction mixture and the reaction mixture is then fed to a reactor, the amine being added through an orifice arranged coaxially to the mixing chamber and the phosgene being added through feed orifices in at least two planes arranged perpendicular to the axis of the mixing chamber.

However, it has been observed that the formation of unwanted by-products can be influenced by the residence time to a certain extend only. In particular, hydrochlorides of amines are still formed in undesired amounts.

It is thus an object of the invention to provide an apparatus and a process for preparing isocyanates by which the formation of unwanted by-products is further reduced while maintaining the positive achievements of the processes known in the art.

The object is achieved by an apparatus for preparing isocyanates according to claim 1 and a process for preparing isocyanates according to claim 10. Advantageous variants of the apparatus are presented in claims 2 to 9.

A first subject of the invention is an apparatus for preparing isocyanates by reacting the corresponding amines with phosgene in the liquid phase, the apparatus comprising
- a mixing chamber for mixing the amine and the phosgene to form a reaction mixture,
- a coaxial inlet penetrating a back plane of the mixing chamber and extending coaxially into the mixing chamber with an orifice opening out into the mixing chamber at a distance L1 to the back plane, and
- radial orifices opening out into the mixing chamber in at least a first plane arranged perpendicular to the axis of the mixing chamber,
wherein all radial orifices are arranged upstream of the orifice of the coaxial inlet in the main flow direction of the reaction mixture.

A second subject of the invention is a process for preparing isocyanates by reacting the corresponding amines with phosgene in the liquid phase, wherein either the amine as a first reactant and the phosgene as a second reactant or the phosgene as a first reactant and the amine as a second reactant are mixed in a mixing chamber to form a reaction mixture and the reaction mixture is fed to a reactor, the first reactant being added through a coaxial inlet penetrating a back plane of the mixing chamber and extending coaxially into the mixing chamber with an orifice opening out into the mixing chamber at a distance L1 to the back plane, and the second reactant being added through radial orifices opening out into the mixing chamber in at least a first plane arranged perpendicular to the axis of the mixing chamber, wherein all radial orifices are arranged upstream of the orifice of the coaxial inlet in the main flow direction of the reaction mixture.

It has been found that positioning the radial orifices upstream of the orifice of the coaxial inlet yields less unwanted by-products like hydrochlorides of amines.

In the context of this invention, the term "upstream" is to be understood in relation to the main direction of flow of the reaction mixture. Thus, "upstream of the orifice of the coaxial inlet" means anywhere in the space between the back plane and the plane perpendicular to the axis of the mixing chamber where the outlet of the orifice is part of that plane. Or, in other words, a plane upstream of the orifice of the coaxial inlet, has a distance from the back plane that is smaller or equal to the distance L1.

In preferred embodiments the apparatus comprises radial orifices opening out into the mixing chamber in a first plane and at least a second plane arranged perpendicular to the axis of the mixing chamber and distant from each other.

Thus, a preferred embodiment of the first subject of the invention is an apparatus for preparing isocyanates by reacting the corresponding amines with phosgene in the liquid phase, the apparatus comprising
- a mixing chamber for mixing the amine and the phosgene to form a reaction mixture,
- a coaxial inlet penetrating a back plane of the mixing chamber and extending coaxially into the mixing chamber with an orifice opening out into the mixing chamber at a distance L1 to the back plane, and
- radial orifices opening out into the mixing chamber in a first plane and at least a second plane arranged perpendicular to the axis of the mixing chamber and distant from each other,
wherein the first plane and the second plane are both arranged upstream of the orifice of the coaxial inlet in the main flow direction of the reaction mixture.

A preferred embodiment of the second subject of the invention is a process for preparing isocyanates by reacting the corresponding amines with phosgene in the liquid phase, wherein either the amine as a first reactant and the phosgene as a second reactant or the phosgene as a first reactant and the amine as a second reactant are mixed in a mixing chamber to form a reaction mixture and the reaction mixture is fed to a reactor, the first reactant being added through a coaxial inlet penetrating a back plane of the mixing chamber and extending coaxially into the mixing chamber with an orifice opening out into the mixing chamber at a distance L1 to the back plane, and the second reactant being added through radial orifices opening out into the mixing chamber in a first plane and at least a second plane arranged perpendicular to the axis of the mixing chamber and distant from each other, wherein the first plane and the second plane are both arranged upstream of the orifice of the coaxial inlet in the main flow direction of the reaction mixture

In the context of the invention, the "first plane" is understood to be the plane located closer to the back plane, and the "second plane" is understood to be the plane located closer to the orifice of the coaxial inlet.

The amine used to prepare isocyanates is, for example, a monoamine, a diamine, a triamine or higher-functionality amine. However, preference is given to using monoamines or diamines. According to the amine used, the corresponding monoisocyanates, diisocyanates, triisocyanates or higher-functionality isocyanates are formed. Preference is given to preparing monoisocyanates or diisocyanates by the process according to the invention.

The amines and isocyanates may be aliphatic, cycloaliphatic or aromatic. Cycloaliphatic isocyanates are those which comprise at least one cycloaliphatic ring system.

Aliphatic isocyanates are those which have exclusively isocyanate groups bonded to straight or branched chains.

Aromatic isocyanates are those which have at least one isocyanate group bonded to at least one aromatic ring system.

In the context of this application, (cyclo)aliphatic isocyanates is a short form of cycloaliphatic and/or aliphatic isocyanates.

Examples of aromatic diisocyanates are monomeric diphenylmethane 2,4'- or 4,4'-diisocyanate (MDI) and higher oligomers thereof (PMDI) or mixtures thereof, toluene 2,4- and/or 2,6-diisocyanate (TDI) and naphthalene 1,5- or 1,8-diisocyanate (NDI).

Preferred (cyclo)aliphatic diisocyanates are those having from 4 to 20 carbon atoms.

Examples of customary aliphatic diisocyanates are tetramethylene 1,4-diisocyanate, hexamethylene diisocyanate (1,6-diisocyanatohexane), octamethylene 1,8-diisocyanate, decamethylene 1,10-diisocyanate, dodecamethylene 1,12-diisocyanate, tetradecamethylene 1,14-diisocyanate, derivatives of lysine diisocyanate, tetramethylxylylene diisocyanate (TMXDI), trimethylhexane diisocyanate or tetramethylhexane diisocyanate, and also 3(or 4),8(or 9)-bis(isocyanatomethyl)tricyclo[5.2.1.02.6]decane isomer mixtures, and cycloaliphatic diisocyanates such as 1,4-, 1,3- or 1,2-diisocyanatocyclohexane, 4,4'- or 2,4'-di(isocyanatocyclohexyl)methane, 1-isocyanato-3,3,5-trimethyl-5-(isocyanatomethyl)cyclohexane (isophorone diisocyanate), 1,3- or 1,4-bis(isocyanatomethyl)cyclohexane, 2,4- or 2,6-diisocyanato-1-methylcyclohexane.

Particular preference is given to MDI/PMDI isomer and oligomer mixtures, and to TDI isomer mixtures.

To prepare monoisocyanates, it is likewise possible to use aliphatic, cycloaliphatic or aromatic amines. A preferred aromatic amine is especially aniline.

The phosgene can be dissolved in an inert solvent before addition to the mixing chamber. Suitable inert solvents in which the phosgene is dissolved are, for example, chlorinated aromatic hydrocarbons, for example monochlorobenzene or dichlorobenzene, or else toluene. The ratio of phosgene to inert solvent is preferably in the range from 1:0 to 1:2, especially in the range from 1:0 to 1:1.

In a preferred embodiment, the amine is fed into the mixing chamber as the first reactant through the coaxial inlet and phosgene is fed into the mixing chamber as the second reactant through the radial orifices perpendicular to the axis of the mixing chamber.

In an alternative preferred embodiment, phosgene is fed into the mixing chamber as the first reactant through the coaxial inlet and the amine is fed into the mixing chamber as the second reactant through the radial orifices perpendicular to the axis of the mixing chamber.

Preferably, the second reactant is added via at least two radial orifices in each of the planes arranged perpendicular to the axis of the mixing chamber. The feed orifices through which the second reactant is added are preferably arranged such that the main directions of the feed orifices meet in the axis of the mixing chamber. By virtue of the arrangement of the feed orifices such that the main directions meet in the axis of the mixing chamber, the second reactant jets added via the feed orifices hit the outer wall of the coaxial inlet and flow towards the outlet of the coaxial inlet. This achieves rapid mixing of the second reactant and the first reactant at the outlet of the coaxial inlet and in a small area downstream of the outlet of the coaxial inlet. This gives rise to a homogeneous distribution of the second reactant in flow direction of the first reactant.

It is also preferred when the feed orifices of the first plane are arranged rotated about the axis of the mixing chamber with respect to the feed orifices of the second plane. It is particularly preferred when the feed orifices, in the case of two feed orifices each per plane, are arranged rotated by 90 degrees with respect to one another.

The mixing chamber in which the first reactant is mixed with the second reactant preferably has a ratio of length to diameter which is in the range from 1 to 2 and especially in the range from 1 to 1.5. The orifice arranged coaxially to the axis of the mixing chamber, through which the first reactant is added, projects into the mixing chamber. To this end, the orifice through which the first reactant is added is configured, for example, as a nozzle. The orifice through which the first reactant is added is then the exit orifice of the nozzle. The ratio of the diameter of the orifice through which the first reactant is added and the diameter of the mixing chamber is preferably in the range from 0.05 to 0.5, more preferably in the range from 0.1 to 0.4 and especially in the range from 0.15 to 0.35.

Preferably, the inside of the coaxial inlet at the orifice is cylindrical and the respective outside is conical with a cone angle (α) in the range from 5° to 30° with an increasing diameter towards the back plane of the mixing chamber. More preferably, the cone angle (α) is in the range from 7° to 15°. By this design, the flow of the first reactant through the outlet of the coaxial inlet and the flow of the second reactant along the outer wall of the coaxial inlet are directed in a favourable manner, promoting a quick and intensive mixing of the reactants.

In this embodiment, the ratio (L4 / D) of the length L4 of the cylindrical part of the coaxial inlet at the orifice to the inner diameter D of the cylindrical part of the coaxial inlet at the orifice is preferably in the range from 1 to 5, more preferably in the range from 2 to 3. It has been found that a ratio (L4 / D) in the preferred ranges represents a good compromise between advantageous flow characteristics, in particular prevention of flow separation at the outlet of the coaxial inlet, and a desired small pressure drop.

Preferably, the transition from the inner surface of the coaxial inlet to the front end of the orifice is curved with an inside radius of curvature (rl) in the range from 0 mm to 10 mm, and the transition from the outer surface of the coaxial inlet to the front end of the orifice is curved with an outside radius of curvature (rA) in the range from 0 mm to 100 mm. More preferably, the inside radius of curvature (rl) is in the range from 0 mm to 2 mm, and the outside radius of curvature (rA) is in the range from 25 mm to 60 mm. It is even more preferred that the inside radius of curvature (rl) is minimized to form a sharp edge. A curved front end of the orifice within the preferred ranges of radii rl and rA ensures a small tip of the orifice enabling high shear mixing of the first reactant and the second reactant without compromising on the mechanical integrity of the outlet of the orifice.

The first plane with radial orifices opening out into the mixing chamber perpendicularly to the axis of the mixing chamber is arranged upstream of the orifice of the coaxial inlet in the main flow direction of the reaction mixture and thus between the back plane of the mixing chamber and the outlet of the coaxial inlet. Preferably, the ratio (L2 / L1) of the distance L2 between the first plane and the orifice of the coaxial inlet to the distance L1 is in the range from 0.1 to 1, more preferably in the range from 0.4 to 0.9, and in particular in the range from 0.6 to 0.8.

In preferred embodiments, a second plane with radial orifices opening out into the mixing chamber perpendicularly to the axis of the mixing chamber is arranged upstream of the orifice of the coaxial inlet in the main flow direction of the reaction mixture between the first plane with radial orifices and the outlet of the coaxial inlet. Preferably, the ratio (L3 / L2) of the distance L3 between the second plane and the orifice of the coaxial inlet to the distance L2 between the first plane and the orifice of the coaxial inlet is in the range from 0 to 0.95, more preferably in the range from 0.1 to 0.9, and in particular in the range from 0.5 to 0.7.

The second reactant is preferably added through feed orifices in a maximum of five planes arranged perpendicular to the axis of the mixing chamber upstream of the orifice of the coaxial inlet in the main flow direction of the reaction mixture. It is more preferred when the second reactant is added through feed orifices in a maximum of three planes arranged perpendicular to the axis of the mixing chamber upstream of the orifice of the coaxial inlet in the main flow direction of the reaction mixture. It is particularly preferred when the second reactant is added through feed orifices in two planes arranged perpendicular to the axis of the mixing chamber.

The ratio of the distance L3 between the second plane and the orifice of the coaxial inlet with respect to the diameter of the mixing chamber is preferably in the range of 0 to 1, more preferably in the range of 0.01 to 0.5 and in particular in the range of 0.05 to 0.2. When the second reactant is added through radial feed orifices in more than one plane perpendicular to the axis of the mixing chamber and located upstream of the orifice of the coaxial inlet through which the first reactant is added in the main direction of flow of the reaction mixture, the above preferred ranges apply to the plane closest to the orifice of the coaxial inlet.

The number of feed orifices in the individual planes is preferably not more than five, more preferably not more than four and especially two. The number of feed orifices in the individual planes gives rise to a good distribution of the second reactant in the mixing chamber. In this context, it is additionally preferred when the orifices of the individual planes are rotated with respect to one another, such that the feed orifices of the individual planes are not aligned in flow direction. In the case of feed orifices in more than two planes, the feed orifices of the individual planes are preferably rotated evenly in relation to each other. The angle by which the individual planes are rotated in relation to each other is preferably calculated to be β = 180° / zo, where β is the angle by which the planes are rotated with respect to one another and z₀ is the number of orifices per plane.

The diameter of the feed orifices through which the second reactant is added is preferably less than the distance of the planes in which the feed orifices are arranged. The diameter of the feed orifices in relation to the diameter of the mixing chamber is preferably in the range from 0.01 to 0.5, more preferably in the range from 0.02 to 0.3 and especially in the range from 0.03 to 0.25.

The radial feed orifices can open into the mixing chamber at any desired angle. The axes of the feed orifices preferably intersect with the axis of the mixing chamber. More preferably, the feed orifices open into the mixing chamber at an angle of 90°.

The feed orifices through which the second reactant is added are preferably nozzle orifices. This means that the second reactant is fed to the mixing chamber through lines and a cross-sectional constriction in the form of a nozzle is formed at the end of the lines. The second reactant then exits from the nozzle into the mixing chamber. The feed orifice of the second reactant is preferably flush with the wall of the mixing chamber. The nozzles may have either circular orifices or orifices deviating from the circular shape.

In a preferred embodiment, the apparatus is composed of at least two sealingly connected components with the coaxial inlet being part of a first component and the radial orifices being part of a second component. This embodiment enables a quick and easy exchange of parts, for example to adjust the apparatus to changing plant loads or a change in reactants which makes it suitable for multipurpose plants.

The mixing chamber in which the first reactant is mixed with the second reactant is preferably rotationally symmetric. When the mixing chamber does not have a circular cross section, the diameter of the mixing chamber always means the hydraulic diameter.

At its downstream end, the mixing chamber preferably has a diameter constriction by virtue of which the reaction mixture is backmixed. The backmixing is effected as a result of the deflection of the flow owing to the diameter constriction.

The diameter constriction at the downstream end of the mixing chamber is preferably configured with an angle in the range from 10° to 80° relative to the axis of the mixing chamber. The diameter constriction at the downstream end is more preferably configured with an angle of from 15° to 60° and especially preferably with an angle of from 18° to 40° relative to the axis of the mixing chamber. The diameter constriction at the downstream end of the mixing chamber is preferably a conical constriction. The ratio of the diameter of the diameter constriction to which the cross section is reduced is, based on the diameter of the mixing chamber, in the range from 0.2 to 0.7, more preferably in the range from 0.25 to 0.65 and especially in the range from 0.3 to 0.6. As well as backmixing, the diameter constriction thus also results in acceleration of the reaction mixture.

The outlet of the orifice of the coaxial inlet may be located in an area of the mixing chamber with or without diameter constriction. In case that the mixing chamber has a diameter constriction at its downstream end, the outlet of the orifice of the coaxial inlet is preferably located in the area with the diameter constriction. The volume of the mixing chamber is the volume up to the end of the constriction, i.e. up to the entry into the zone with constant cross section which follows the mixing chamber downstream. The volume of the coaxial inlet which projects into the mixing chamber is not part of the volume of the mixing chamber.

For flow homogenization, the diameter constriction is preferably followed downstream by a zone with a constant diameter in which there is only minor backmixing.

The ratio of the length of the zone with constant diameter and the diameter of this zone is preferably in the range from 1 to 10, more preferably in the range from 1.5 to 9 and especially in the range from 2 to 8.

The zone with constant diameter is preferably followed downstream by a zone with a cross-sectional enlargement, the cross-sectional enlargement having an opening angle based on the axis of the zone at which there is no discontinuity of flow. This means that the cross-sectional enlargement is configured in the form of a diffuser. The cross-sectional enlargement widens the diameter until the diameter of the reactor which is preferably configured as a tubular reactor is attained. In this context, it is possible that the diameter is widened stepwise, in which case a region with constant diameter is arranged between each of the individual stages in which the diameter is widened.

In order to prevent discontinuity of flow, the opening angle of the cross-sectional enlargement relative to the axis of the zone is preferably less than 15°, more preferably less than 10° and especially preferably less than 8°.

For the first variant of the second subject of the invention where the amine is fed into the mixing chamber as the first reactant through the coaxial inlet and phosgene is fed into the mixing chamber as the second reactant through the radial orifices perpendicular to the axis of the mixing chamber, the following clauses present preferred embodiments of this first variant of the second subject of the invention.

### Clause A1:

A process for preparing isocyanates by reacting the corresponding amines with phosgene in the liquid phase, wherein the amine as a first reactant and the phosgene as a second reactant are mixed in a mixing chamber to form a reaction mixture and the reaction mixture is fed to a reactor, the amine being added through a coaxial inlet penetrating a back plane of the mixing chamber and extending coaxially into the mixing chamber with an orifice opening out into the mixing chamber at a distance L1 to the back plane, and the phosgene being added through radial orifices opening out into the mixing chamber in at least a first plane arranged perpendicular to the axis of the mixing chamber, wherein all radial orifices are arranged upstream of the orifice of the coaxial inlet in the main flow direction of the reaction mixture.

### Clause A2:

The process according to clause A1, wherein the ratio (L2 / L1) of the distance L2 between the first plane (6) and the orifice (4) of the coaxial inlet (3) to the distance L1 is from 0.1 to 1.

### Clause A3:

The process according to clause A1 or A2, wherein the phosgene is added through radial orifices opening out into the mixing chamber in a first plane and at least a second plane arranged perpendicular to the axis of the mixing chamber and distant from each other.

### Clause A4:

The process according to clause A3, wherein the ratio (L3 / L2) of the distance L3 between the second plane (7) and the orifice (4) of the coaxial inlet (3) to the distance L2 between the first plane (6) and the orifice (4) of the coaxial inlet (3) is from 0 to 0.95.

### Clause A5:

The process according to any one of clauses A1 to A4, wherein the inside of the coaxial inlet (3) at the orifice (4) is cylindrical and the respective outside is conical with a cone angle (α) from 10° to 30° with an increasing diameter towards the back plane (2) of the mixing chamber (1).

### Clause A6:

The process according to clause A5, wherein the ratio (L4 / D) of the length L4 of the cylindrical part of the coaxial inlet (3) at the orifice (4) to the inner diameter D of the cylindrical part of the coaxial inlet (3) at the orifice (4) is from 1 to 5, preferably from 2 to 3.

### Clause A7:

The process according to any one of clauses A1 to A6, wherein the transition from the inner surface of the coaxial inlet (3) to the front end of the orifice (4) is curved with an inside radius of curvature (rl) in the range from 0 mm to 10 mm, and the transition from the outer surface of the coaxial inlet (3) to the front end of the orifice (4) is curved with an outside radius of curvature (rA) in the range from 0 mm 100 mm.

### Clause A8:

The process of clause A7, wherein the inside radius of curvature (rl) is minimized to form a sharp edge.

### Clause A9:

The process of any one of clauses A1 to A8, wherein the apparatus is composed of at least two sealingly connected components with the coaxial inlet (3) being part of a first component and the radial orifices (5) being part of a second component.

For the second variant of the second subject of the invention where phosgene is fed into the mixing chamber as the first reactant through the coaxial inlet and the amine is fed into the mixing chamber as the second reactant through the radial orifices perpendicular to the axis of the mixing chamber, the following clauses present preferred embodiments of this second variant of the second subject of the invention.

### Clause B1:

A process for preparing isocyanates by reacting the corresponding amines with phosgene in the liquid phase, wherein the phosgene as a first reactant and the amine as a second reactant are mixed in a mixing chamber to form a reaction mixture and the reaction mixture is fed to a reactor, the phosgene being added through a coaxial inlet penetrating a back plane of the mixing chamber and extending coaxially into the mixing chamber with an orifice opening out into the mixing chamber at a distance L1 to the back plane, and the amine being added through radial orifices opening out into the mixing chamber in at least a first plane arranged perpendicular to the axis of the mixing chamber, wherein all radial orifices are arranged upstream of the orifice of the coaxial inlet in the main flow direction of the reaction mixture.

### Clause B2:

The process according to clause B1, wherein the ratio (L2 / L1) of the distance L2 between the first plane (6) and the orifice (4) of the coaxial inlet (3) to the distance L1 is from 0.1 to 1.

### Clause B3:

The process according to clause B1 or B2, wherein the amine is added through radial orifices opening out into the mixing chamber in a first plane and at least a second plane arranged perpendicular to the axis of the mixing chamber and distant from each other.

### Clause B4:

The process according to clause B3, wherein the ratio (L3 / L2) of the distance L3 between the second plane (7) and the orifice (4) of the coaxial inlet (3) to the distance L2 between the first plane (6) and the orifice (4) of the coaxial inlet (3) is from 0 to 0.95.

### Clause B5:

The process according to any one of clauses B1 to B4, wherein the inside of the coaxial inlet (3) at the orifice (4) is cylindrical and the respective outside is conical with a cone angle (α) from 10° to 30° with an increasing diameter towards the back plane (2) of the mixing chamber (1).

### Clause B6:

The process according to clause B5, wherein the ratio (L4 / D) of the length L4 of the cylindrical part of the coaxial inlet (3) at the orifice (4) to the inner diameter D of the cylindrical part of the coaxial inlet (3) at the orifice (4) is from 1 to 5, preferably from 2 to 3.

### Clause B7:

The process according to any one of clauses B1 to B6, wherein the transition from the inner surface of the coaxial inlet (3) to the front end of the orifice (4) is curved with an inside radius of curvature (rl) in the range from 0 mm to 10 mm, and the transition from the outer surface of the coaxial inlet (3) to the front end of the orifice (4) is curved with an outside radius of curvature (rA) in the range from 0 mm to 100 mm.

### Clause B8:

The process of clause B7, wherein the inside radius of curvature (rl) is minimized to form a sharp edge.

### Clause B9:

The process of any one of clauses B1 to B8, wherein the apparatus is composed of at least two sealingly connected components with the coaxial inlet (3) being part of a first component and the radial orifices (5) being part of a second component.

The invention is explained in more detail below with reference to the drawings. The drawings are to be interpreted as in-principle presentation. They do not constitute any restriction of the invention, for example with regards to specific dimensions or design variants. In the figures:
- Fig. 1: shows a mixing chamber for an apparatus for preparing isocyanates as a first embodiment of the invention.
- Fig. 2: shows an orifice of a coaxial inlet into the mixing chamber of Fig. 1 in more detail.
- Fig. 3: shows a more detailed view of the edge of the orifice of Fig. 2.

### List of reference numerals used:

- 1: mixing chamber
- 2: back plane of mixing chamber
- 3: coaxial inlet
- 4: orifice of coaxial inlet
- 5: radial orifices
- 6: first plane
- 7: second plane
- 8: axis of the mixing chamber
- D: diameter of the cylindrical part of the coaxial inlet at the orifice 4
- L1: distance between orifice 4 and back plane 2
- L2: distance between orifice 4 and first plane 6
- L3: distance between orifice 4 and second plane 7
- L4: length of the cylindrical part of the coaxial inlet at the orifice 4
- rA: outside radius of curvature of the front end of the orifice 4
- rl: inside radius of curvature of the front end of the orifice 4
- α: cone angle at the outlet of the orifice 4

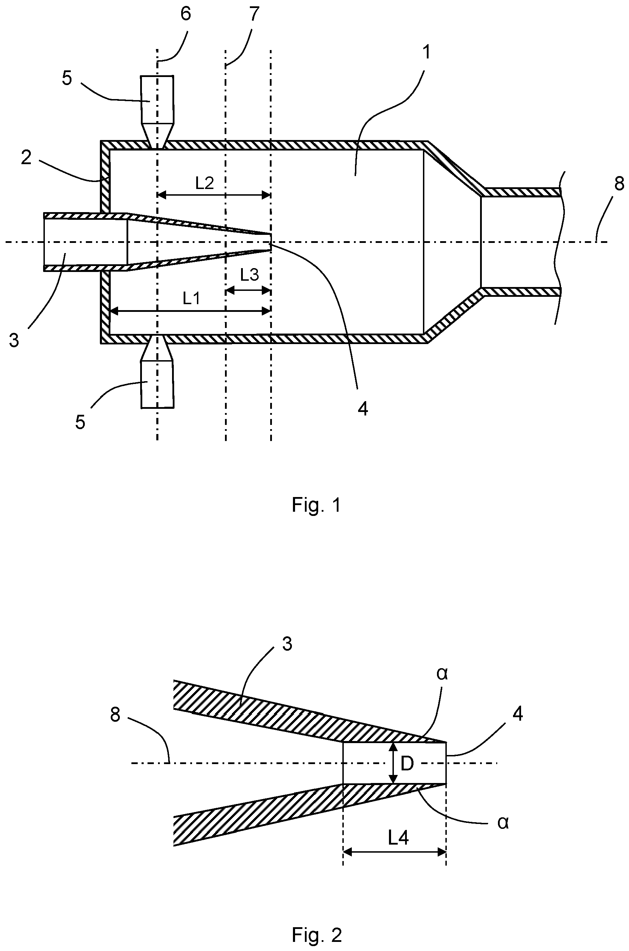

Fig. 1 shows a longitudinal cut view of a mixing chamber for an apparatus for preparing isocyanates by reacting the corresponding amines with phosgene in the liquid phase as a first embodiment of the invention.

In processes for preparing isocyanates by reacting the corresponding amines with phosgene in the liquid phase, the amine is mixed with the phosgene, before the mixed reactants are fed to a reactor in which the reaction takes place.

The apparatus comprises a mixing chamber 1 for mixing the amine and the phosgene to form a reaction mixture. The amine is preferably added through an orifice 4 of an inlet 3 which is arranged coaxially to the mixing chamber 1. Alternatively, it is also possible that the phosgene is supplied through the coaxial inlet 3 to the mixing chamber. However, preference is given to adding the amine through the coaxial inlet 3. The orifice 4 of the coaxial inlet 3 may have any form or design suitable for feeding the amine or the phosgene into the mixing chamber. In the example shown in Fig. 1, the orifice 4 is configured in the form of a nozzle which projects into the mixing chamber 1. The coaxial inlet 3 penetrates a back plane 2 of the mixing chamber 1 and extends coaxially into the mixing chamber 1 with the orifice 4 opening out into the mixing chamber 1 at a distance L1 to the back plane 2.

The apparatus further comprises radial orifices 5 through which the phosgene or, in the case of addition of the phosgene through the coaxial inlet 3 the amine, is added. The feed orifices 5 are likewise preferably configured as nozzles. In the example shown in Fig. 1, the radial orifices 5 are arranged in two planes 6, 7 arranged perpendicular to the axis 8 of the mixing chamber 1 upstream of the orifice 4 of the coaxial inlet 3 in the main flow direction of the reaction mixture. The planes 6, 7 are indicated in Fig. 1 by dash-dotted lines. The first plane 6 is arranged closer towards the back plane 2 of the mixing chamber 1, whereas the second plane 7 is arranged closer towards the outlet of the orifice 4 of the coaxial inlet 3. In addition to the embodiment shown, it is also possible that the radial orifices 5 are arranged in more than two planes.

In the example shown, two radial orifices 5 are arranged in each plane 6, 7, whereby the orifices 5 are arranged diametrically opposite each other. Due to that arrangement, the main directions of the radial orifices 5 meet in the axis 8 of the mixing chamber 1. The orifices 5 of the first plane 6 are arranged rotated by 90° about the axis 8 of the mixing chamber with respect to the orifices 5 of the second plane 7. The radial orifices of the second plane 6 are located in front of and behind the coaxial inlet 3 in relation to the cutting plane of Fig. 1 and are thus not shown in Fig. 1.

The first plane 6 is arranged at a distance L2 from the orifice 4 of the coaxial inlet 3. The second plane 7 is arranged at a distance L3 from the orifice 4 of the coaxial inlet 3. In the example shown, the ratio (L2 / L1) of the distance L2 to the distance L1 is about two third, i.e. 0.66. The ratio (L3 / L2) of the distance L3 to the distance L2 is about 0.45.

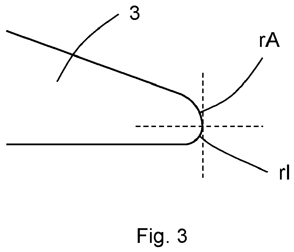

Fig. 2 shows the orifice 4 of the coaxial inlet 3 into the mixing chamber of Fig. 1 in more detail. Fig. 3 shows a more detailed view of the edge of the orifice of Fig. 2. Compared to Fig. 2, only the upper part of the rotationally symmetric orifice is shown.

The inside of the coaxial inlet 3 at the orifice 4 is cylindrical and the respective outside is conical. The cone angle α between the inside and the outside of the orifice 4 at its end is 18° in the example shown. The diameter of the cone increases towards the back plane 2 of the mixing chamber 1.

The ratio (L4 / D) of the length L4 of the cylindrical part of the coaxial inlet 3 at the orifice 4 to the inner diameter D of the cylindrical part of the coaxial inlet 3 at the orifice 4 is about 2.

As illustrated in Fig. 3, the transition from the inner surface of the coaxial inlet 3 to the front end of the orifice 4 is curved with an inside radius of curvature (rl), and the transition from the outer surface of the coaxial inlet 3 to the front end of the orifice 4 is curved with an outside radius of curvature (rA) which in this example is larger than the inside radius of curvature (rl). Depending on the material from which the coaxial inlet 3 is formed and depending on the respective manufacturing capabilities, the inside radius of curvature (rl) is minimized to form a sharp edge.

### Comparative example

For the production of toluylene diisocyanate (TDI) by reacting toluylenediamine (TDA) with phosgene in the liquid phase using monochlorobenzene (MCB) as solvent, an apparatus comprising a mixing chamber was used. The principle design of the mixing chamber was similar to that shown in Fig. 1.

The amine TDA was fed as a first reactant through a coaxial inlet 3. The end of the coaxial inlet 3 was formed by a nozzle as the orifice 4 with a distance L1 between the orifice 4 and the back plane 2.

For the supply of phosgene as a second reactant, two radial orifices 5 were arranged in a first plane 6 and two radial orifices 5 were arranged in a second plane 7. The orifices 5 were arranged diametrically opposite each other. The orifices 5 of the first plane 6 were arranged rotated by 90° about the axis 8 of the mixing chamber with respect to the orifices 5 of the second plane 7. The feed orifices 5 were configured as nozzles at the end of respective feed lines.

The first plane 6 was arranged upstream of the orifice 4 of the coaxial inlet 3 at a distance L2 from the orifice 4. The second plane 7 was arranged downstream of the orifice 4 of the coaxial inlet 3 at a distance L3 from the orifice 4. The ratio (L2 / L1) of the distance L2 to the distance L1 was 0.23, and the ratio (L3 / L2) of the distance L3 to the distance L2 was -1, the negative sign (-) resulting from the fact that the second plane 7 was arranged downstream of the orifice 4 of the coaxial inlet 3.

The mixing chamber 1 had a conical constriction, the diameter decreasing from the mixing chamber diameter to the exit diameter. The mixing chamber was followed downstream by a zone of constant diameter. The zone of constant diameter was followed downstream by an enlargement where the diameter increased to the diameter of a downstream tubular reactor.

TDA as the first reactant was fed through the coaxial inlet 3 as a mixed stream of 12.1 t/h (metric tons per hour) of TDA and 50 t/h of monochlorobenzene. The phosgene-containing second reactant was fed into the mixing chamber 1 through the four radial orifices 5 in a total amount of 140.2 t/h. The composition of the second reactant was 91 wt.-% of phosgene and 9 wt.-% of monochlorobenzene.

In order to ensure the product quality, the reaction product was analysed for unwanted by-products after the tubular reactor. One of the major by-products of the phosgenation reaction are hydrochlorides of the TDA feed, namely the dihydrochlorides of 2,4-TDA and 2,6-TDA (TDA*2HCl). Amine dihydrochlorides are hardly soluble in the reaction mixture and cause erosion. As they are being phosgenated very slowly, they decompose downstream to amines which react with isocyanates to form ureas which further react to unwanted by-products that may cause fouling or blocking of distillation column internals for example.

The average particle concentrations of TDA*2HCl was determined to 0.26 vol-% with an average particle diameter of 56 µm. The yield of the wanted product TDI was 96.9 %.

### Example according to the invention

The mixing chamber 1 of the plant for the production of TDI according to the Comparative Example was modified. The first plane 6 was arranged upstream of the orifice 4 of the coaxial inlet 3 at a distance L2 from the orifice 4. The second plane 7 was arranged upstream of the orifice 4 of the coaxial inlet 3 as well at a distance L3 from the orifice 4. The ratio (L2 / L1) of the distance L2 to the distance L1 was 0.75, and the ratio (L3 / L2) of the distance L3 to the distance L2 was 0.6.

The inside of the coaxial inlet 3 at the orifice 4 was cylindrical and the respective outside was conical with a cone angle (α) of 10° with an increasing diameter towards the back plane 2 of the mixing chamber 1. The transition from the inner surface of the coaxial inlet 3 to the front end of the orifice 4 was curved with an inside radius of curvature (rl) of 0 mm, thus it was designed as a sharp edge. The transition from the outer surface of the coaxial inlet 3 to the front end of the orifice 4 was curved with an outside radius of curvature (rA) of 50 mm.

For the same process parameters as in the Comparative Example, different results in view of the unwanted by-products were obtained. The average particle concentrations of TDA*2HCl was determined to 0.04 vol-% with an average particle diameter of 40 µm. The yield of the wanted product TDI was 96.7 %.

Thus, by choosing the distances between the radial orifices and the orifice of the coaxial inlet in a range according to the invention, the particle concentration of the unwanted by-product TDA*2HCl could be reduced by 85 % from 0.26 vol.-% to only 0.04 vol.-% without compromising on the TDI yield.

## Claims

1. An apparatus for preparing isocyanates by reacting the corresponding amines with phosgene in the liquid phase, the apparatus comprising
- a mixing chamber (1) for mixing the amine and the phosgene to form a reaction mixture,
- a coaxial inlet (3) penetrating a back plane (2) of the mixing chamber (1) and extending coaxially into the mixing chamber (1) with an orifice (4) opening out into the mixing chamber (1) at a distance L1 to the back plane (2), and
- radial orifices (5) opening out into the mixing chamber (1) in at least a first plane (6) arranged perpendicular to the axis (8) of the mixing chamber (1),
**characterized in that** all radial orifices (5) are arranged upstream of the orifice (4) of the coaxial inlet (3) in the main flow direction of the reaction mixture.

2. The apparatus according to claim 1, wherein the ratio (L2 / L1) of the distance L2 between the first plane (6) and the orifice (4) of the coaxial inlet (3) to the distance L1 is from 0.1 to 1.

3. The apparatus according to claim 1 or 2, wherein the apparatus comprises radial orifices (5) opening out into the mixing chamber (1) in a first plane (6) and at least a second plane (7) arranged perpendicular to the axis (8) of the mixing chamber (1) and distant from each other.

4. The apparatus according to claim 3, wherein the ratio (L3 / L2) of the distance L3 between the second plane (7) and the orifice (4) of the coaxial inlet (3) to the distance L2 between the first plane (6) and the orifice (4) of the coaxial inlet (3) is from 0 to 0.95.

5. The apparatus according to any one of claims 1 to 4, wherein the inside of the coaxial inlet (3) at the orifice (4) is cylindrical and the respective outside is conical with a cone angle (α) from 5° to 30° with an increasing diameter towards the back plane (2) of the mixing chamber (1).

6. The apparatus according to claim 5, wherein the ratio (L4 / D) of the length L4 of the cylindrical part of the coaxial inlet (3) at the orifice (4) to the inner diameter D of the cylindrical part of the coaxial inlet (3) at the orifice (4) is from 1 to 5, preferably from 2 to 3.

7. The apparatus according to any one of claims 1 to 6, wherein the transition from the inner surface of the coaxial inlet (3) to the front end of the orifice (4) is curved with an inside radius of curvature (rl) in the range from 0 mm to 10 mm, and the transition from the outer surface of the coaxial inlet (3) to the front end of the orifice (4) is curved with an outside radius of curvature (rA) in the range from 0 mm to 100 mm.

8. The apparatus of claim 7, wherein the inside radius of curvature (rl) is minimized to form a sharp edge.

9. The apparatus of any one of claims 1 to 8, wherein the apparatus is composed of at least two sealingly connected components with the coaxial inlet (3) being part of a first component and the radial orifices (5) being part of a second component.

10. A process for preparing isocyanates by reacting the corresponding amines with phosgene in the liquid phase, wherein either the amine as a first reactant and the phosgene as a second reactant or the phosgene as a first reactant and the amine as a second reactant are mixed in a mixing chamber (1) to form a reaction mixture and the reaction mixture is fed to a reactor, the first reactant being added through a coaxial inlet (3) penetrating a back plane (2) of the mixing chamber (1) and extending coaxially into the mixing chamber (1) with an orifice (4) opening out into the mixing chamber (1) at a distance L1 to the back plane (2), and the second reactant being added through radial orifices (5) opening out into the mixing chamber (1) in at least a first plane (6) arranged perpendicular to the axis (8) of the mixing chamber (1), **characterized in that** all radial orifices (5) are arranged upstream of the orifice (4) of the coaxial inlet (3) in the main flow direction of the reaction mixture.
